# EUROPEAN PATENT APPLICATION

(11) **EP 3 878 362 A1**
(43) Date of publication of application: **15.09.2021**
(21) Application number: 21161026.6
(22) Date of filing: 05.03.2021
(51) Int. Cl.: A61B 5/11, G16H 10/60

(54) **BED EXIT PREDICTION BASED ON PATIENT BEHAVIOR PATTERNS**

(30) Priority: 11.03.2020 US 202062987999 P
(71) Applicant: Hill-Rom Services, Inc., Batesville, IN 47006-9167 (US)
(72) Inventor: BHAI, Aziz A, Batesville, IN Indiana 47006-9167 (US); COMPARONE, Nicholas, Batesville, IN Indiana 47006-9167 (US); CHRISTIE, John D, Batesville, IN Indiana 47006-9167 (US); GOEWERT, John, Batesville, IN Indiana 47006-9167 (US); WEIDMAN, Bryan, Batesville, IN Indiana 47006-9167 (US); BYERS, John G, Batesville, IN Indiana 47006-9167 (US); OJHA, Unnati, Batesville, IN Indiana 47006-9167 (US); BENZ, Eric D, Batesville, IN Indiana 47006-9167 (US)
(74) Representative: Findlay, Alice Rosemary

(57) **Abstract**

A system for inferring a patient's future behavior may include a patient support apparatus. A data acquisition system may track data related to patient bed exits from the patient support apparatus. A controller may be in communication with the patient support apparatus. The controller may include a processor and a non-transitory memory device. The memory device may include instructions that, when executed by the processor, acquire data related to patient bed exits.

## Description

The present disclosure is related to a patient support apparatus that can predict patient bed exits. More specifically, the present disclosure is related to a patient support apparatus that includes a control system that can predict patient bed exit, displays information related to the exit on a user interface, and alerts the caregiver.

The mobility of a person supported on a patient support apparatus is of interest to caregivers in assessing the risk of the patient making unassisted bed exits. When making an unassisted bed exit, the patient may be at risk for falling and subsequent injury. Many devices, including bed exit alarms, real-time locating systems, and cameras may be used to monitor when a patient exits the bed.

The present disclosure includes one or more of the following features alone or in any combination.

In a first aspect of the disclosed embodiments, a method for inferring a patient's future behavior may include acquiring data related to patient bed exits. The method may also include analyzing the data related to patient bed exits to detect patterns in patient bed exit behavior. The method may also include building a customized schedule of patient bed exit behavior to predicts future patient bed exits. The method may also include notifying a caregiver when a future patient bed exit is to occur.

In some embodiments of the first aspect, the method may include acquiring data related to patient bed exits includes acquiring data from a sensor in a patient support apparatus. The method may also include acquiring data related to patient bed exits includes acquiring data from a real time locating system. The method may also include acquiring data related to patient bed exits includes acquiring data from a camera in a patient room. The method may also include acquiring data related to patient bed exits includes acquiring data related to the patient's medical schedule. The method may also include acquiring data related to patient bed exits includes acquiring data related to the patient's mealtime schedule. The method may also include acquiring data related to patient bed exits includes acquiring data related to the patient's visiting hours schedule.

It may be desired in the first aspect that the method includes combining historical data from similar patients to the data related to patient bed exits to determine a model that predicts future patient bed exits. The method may also include notifying a caregiver when a future patient bed exit is to occur further comprises notifying a caregiver a predetermined time before the future patient bed exit is to occur. The method may also include comparing a patient's current behavior to a patient's predicted behavior. The method may also include updating the customized schedule based on differences between a patient's current behavior and a patient's predicted behavior. The method may also include notifying a caregiver when a future patient bed exit is to occur further comprises notifying the caregiver before the patient wakes up. The method may also include notifying a caregiver when a future patient bed exit is to occur further comprises notifying the caregiver before the patient uses the restroom.

In a second aspect of the disclosed embodiments, a system for inferring a patient's future behavior may include a patient support apparatus. A data acquisition system may track data related to patient bed exits from the patient support apparatus. A controller may be in communication with the patient support apparatus. The controller may include a processor and a non-transitory memory device. The memory device may include instructions that, when executed by the processor, acquire data related to patient bed exits, analyze the data related to patient bed exits to detect patterns in patient bed exit behavior, build a customized schedule of patient bed exit behavior to predicts future patient bed exits, and notify a caregiver when a future patient bed exit is to occur.

In some embodiments of the second aspect, the data acquisition system may include a sensor in a patient support apparatus. The data acquisition system may include a real time locating system. The data acquisition system may include a camera in a patient room. The data related to patient bed exits includes data related to the patient's medical schedule. The data related to patient bed exits may include data related to the patient's mealtime schedule. The data related to patient bed exits may include data related to the patient's visiting hours schedule.

It may be desired in the second aspect that historical data from similar patients is compared to the data related to patient bed exits to determine a model that predicts future patient bed exits. The caregiver may be notified a predetermined time before the future patient bed exit is to occur. A patient's current behavior may be compared to a patient's predicted behavior to update the customized schedule. The caregiver may be notified before the patient wakes up. The caregiver may be notified before the patient uses the restroom.

The invention will now be further described by way of example with reference to the accompanying drawings, in which:
Fig. 1 is a schematic showing the interaction between a patient support apparatus and a hospital information system;
Fig. 2 is a flowchart showing the different steps performed to determine patient behavior; and
Fig. 3 is a simplified schematic showing the model creation for determining patient behavior.

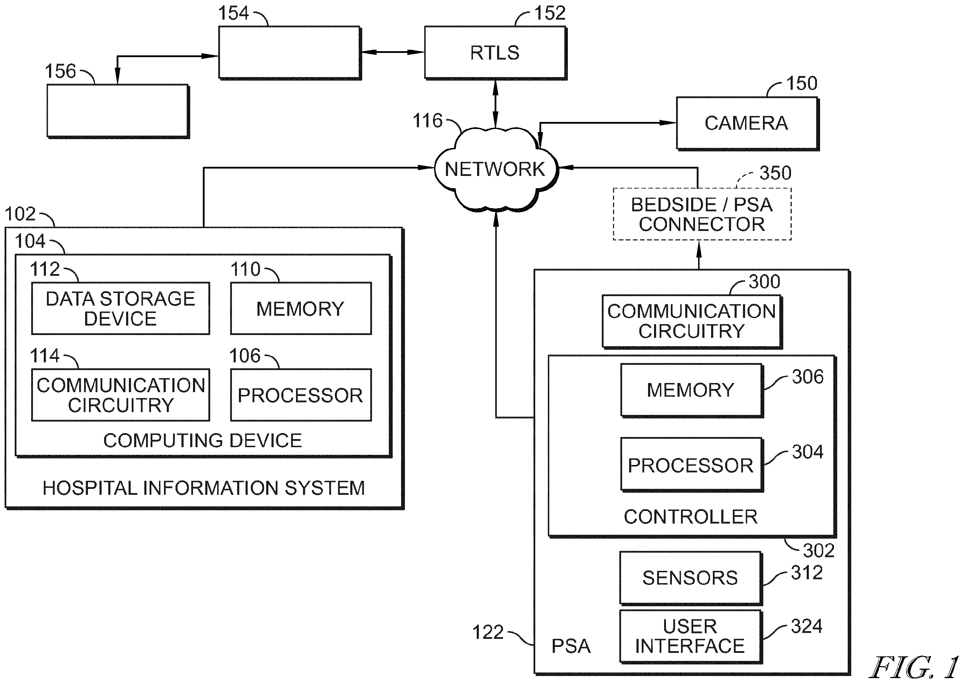

Referring to Fig. 1, a system 100 for a healthcare facility includes a patient support apparatus 122, such as a hospital bed that includes a patient support structure such as a frame that supports a surface or mattress. Thus, according to this disclosure a bed frame, a mattress or both are examples of things considered to be within the scope of the term "patient support structure." However, this disclosure is applicable to other types of patient support apparatuses and other patient support structures, including other types of beds, surgical tables, examination tables, stretchers, and the like.

The patient support apparatus 122 includes a plurality of sensors 312 that are used to determine the patient's mobility score. In some embodiments, these sensors may be load cells. In some embodiments, these sensors maybe air pressure bladders. In some embodiments, these sensors may be contact sensors. In some embodiments, these sensors maybe force sensing resistors. In some embodiments, a patient support apparatus may have multiple such sensors. The sensors 312 are utilized to detect bed exit events by monitoring a pressure on the patient support apparatus 122 and determining when the pressure is removed, which is indicative of the patient exiting the bed. The sensors 312 may also be utilized to monitor when a patient is positioned on a side of the patient support apparatus 122 and at risk for falling.

As shown in Fig. 1, in one embodiment, the patient support apparatus 122, includes communication circuitry 300, a controller 302, and an interface 324. The controller 302 is capable of controlling operational functionality of the patient support apparatus 122 and/or interpreting data signals from the various sensors 312. The communication circuitry 300 is capable of establishing connections and facilitating communications to and from the patient support apparatus 122. The controller 302 is further configured to provide, or relay, status indications to a remote location, such as the nurse call system, via the communication circuitry 300. The status indications may include any type of indication of a component, or a patient relative to a component, of the patient support apparatus 122. The communication circuitry 300 may be embodied as any communication circuit, device, or collection thereof, capable of enabling communications over a network 116 between the patient support apparatus 122 and a hospital information system 102. The communication circuitry 300 may be configured to use any one or more communication technologies (e.g., wired or wireless communications) and associated protocols (e.g., Ethernet, Bluetooth®, Zigbee®, Wi-Fi®, WiMAX, etc.) to effect such communication.

In some embodiments, the patient support apparatus is connected to a bedside/patient support apparatus connector 350 via the communications circuitry 300. The bedside/patient support apparatus connector may have a computing device and server to connect to the network 116.

The controller 302 is connected to various sensors capable of being monitored and interpreted by the controller 302, and various actuators capable of being controlled by the controller 302. The controller 302 is configured to receive data (i.e., electrical signals) from the various sensors and components of the patient support apparatus 122, and control the operation of the components of the patient support apparatus 122 relative to the received data, as is known in the art. To do so, the controller 302 includes a number of electronic components commonly associated with controllers utilized in the control of electromechanical systems. For example, the controller 302 may include, amongst other components customarily included in such devices, a processor 304 and a memory device 306. The memory device 306 may be, for example, a programmable read-only memory device ("PROM") including erasable PROM's (EPROM's or EEPROM's). In use, the memory device 306 is capable of storing, amongst other things, instructions in the form of, for example, a software routine (or routines) which, when executed by the processor 304, allow the controller 302 to control operation of the features of the patient support apparatus 122.

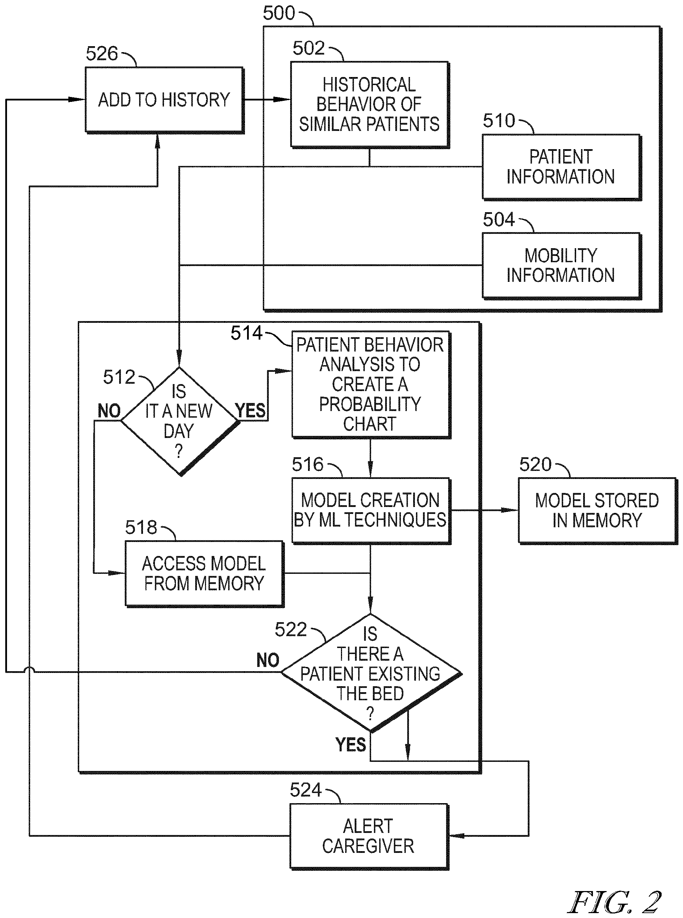

The system 100 includes a hospital information system 102 of one or more hospitals communicatively coupled over a network 116 to various care assets, such as a patient support apparatus 122. To facilitate the transfer of data and other network communications across the hospital information system 102, the hospital information system 102 includes a number of computing devices 104. Each of the computing devices 104 may be embodied as any type of computation or computer device capable of performing the functions described herein, including, without limitation, a server (e.g., stand-alone, rack-mounted, blade, etc.), a network appliance (e.g., physical or virtual), a high-performance computing device, a web appliance, a distributed computing system, a computer, a processor-based system, a multiprocessor system, a smartphone, a tablet computer, a laptop computer, a notebook computer, and/or a mobile computing device. The illustrative computing device 104 of Fig. 2 includes a processor 106 and a memory 110. Of course, the computing device 104 may include additional and/or alternative components, such as those commonly found in a computer (e.g., various input/output devices), in other embodiments. Additionally, in some embodiments, one or more of the illustrative components may be incorporated in, or otherwise form a portion of, another component. For example, the memory 110, or portions thereof, may be incorporated in the processor 106 in some embodiments.

The processor 106 may be embodied as any type of processor capable of performing the functions described herein. For example, the processor 106 may be embodied as a single or multi-core processor(s), digital signal processor, microcontroller, or other processor or processing/controlling circuit. The memory 110 may be embodied as any type of volatile or non-volatile memory or data storage capable of performing the functions described herein. In operation, the memory 110 may store various data and software used during operation of the computing device 104 such as operating systems, applications, programs, libraries, and drivers. The memory 110 is communicatively coupled to the processor 106 via a I/O subsystem, which may be embodied as circuitry and/or components to facilitate input/output operations with the processor 106, the memory 110, and other components of the computing device 104. For example, the I/O subsystem may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations.

A data storage device 112 may be embodied as any type of device or devices configured for short-term or long-term storage of data such as, for example, memory devices and circuits, memory cards, hard disk drives, solid-state drives, or other data storage devices. In use, as described below, the data storage device 112 and/or the memory 110 may store security monitoring policies, configuration policies, or other, similar data. Communication circuitry 114 may be embodied as any communication circuit, device, or collection thereof, capable of enabling communications between the computing devices 104 and/or between one of the computing devices 104 and the patient support apparatus 122. The communication circuitry 114 may be configured to use any one or more communication technology (e.g., wired or wireless communications) and associated protocols (e.g., Ethernet, Bluetooth®, Wi-Fi®, WiMAX, etc.) to effect such communication.

The computing devices 104 of the hospital information system 102 may be configured into separate subsystems for managing data and coordinating communications throughout the hospital information system 102.

The network 116 may be embodied as any type of wired or wireless communication network, including cellular networks (e.g., Global System for Mobile Communications (GSM), 3G, Long Term Evolution (LTE), Worldwide Interoperability for Microwave Access (WiMAX), etc.), digital subscriber line (DSL) networks, cable networks (e.g., coaxial networks, fiber networks, etc.), telephony networks, local area networks (LANs) or wide area networks (WANs), global networks (e.g., the Internet), or any combination thereof. As previously described, at least a portion the patient support apparatuses 122 may be in communication with the hospital information system 102 over the network 116. Accordingly, the network 116 may include any number of network devices (e.g., access points, routers, switches, servers, etc.) as needed to facilitate communications between the hospital information system 102 and the patient support apparatus 122.

Referring still to Fig. 1, a real-time locating system (RTLS) 152 is provided to track the whereabouts of the patient. RTLS 152 includes a patient tag 154 worn by the patient and in communication with a multitude of transceivers 156. Transceivers 156 may be dispersed throughout the patient room. Tag 154 and transceiver 156 each include a housing that contains associated circuitry. The circuitry of tag 154 and transceiver 156 includes for example a processor such as a microprocessor or microcontroller or the like, memory for storing software, and communications circuitry including a transmitter, a receiver and at least one antenna, for example. Tag 154 also includes structure to enable attachment to the patient. For example, tag 154 may include a necklace so that a caregiver can wear the tag 154 around their neck or may include a clip so that the caregiver can attach the tag 154 to their clothing. The tag 154 may include a wristband so that the tag 154 can be worn on the wrists of the associated patients. Transceivers 156 each include mounting hardware, such as brackets or plates or the like, in some embodiments, to permit the transceivers 156 to be mounted at fixed locations in the patient room with fasteners such as screws or the like.

Transceiver 156 communicates wirelessly with tag 154 using radio frequency (RF). According to this disclosure, system 152 operates as a high-accuracy locating system which is able to determine the location of the tag 154 within one foot (30.48 cm) or less of the tag's actual location. System 152 is operable to determine the location of the tag 154 in 2-dimensional space. One example of a high-accuracy locating system contemplated by this disclosure is an ultra-wideband (UWB) locating system. UWB locating systems operate within the 3.1 gigahertz (GHz) to 10.6 GHz frequency range. Accordingly, the tag 154 is tracked by the RTLS 152 to monitor when the patient has left the patient support apparatus 122. Data related to movement of the patient is transmitted over the network 116 to the hospital information system 102.

Additionally, cameras 150 may be positioned in the patient room and in communication with the network 116. As such, the cameras 150 are utilized to monitor when the patient exits the beds. That is time-stamped video of the patient is captured by the camera 150 and transmitted to the hospital information system 102, where the video is monitored for bed exits. In some embodiments, the processor 106 may operate instructions to track bed exit behavior in the video feeds. Therefore, data from the sensors 312, the RTLS 152, and the cameras 150 may all be utilized or individually utilized to track when the patient exits the bed. As set forth below, this data may be used to determine a bed exit schedule for the patient.

In the illustrated embodiment, the processor 106 of the hospital information system 102 can access information gathered through the hospital information system from the memory 110. In some embodiments, the processor can access the current patient's daily routine. In some embodiments, the processor can access the current patient's medical schedule. In some embodiments, the processor can access the current patient's visiting hours. In some embodiments, the processor can access the historical behavior of similar patients. The processor 106 can access mobility information of the current patient from the memory 110 of the patient support apparatus the memory 122 over the network 166. The processor 106 in a computing device 104 of the hospital information system 102 uses the current patient's information, the patient's mobility score, and the historical information from similar patients to perform analysis to determine the patient's behavior patterns. In some embodiments, the processor 304 of the patient support device 122 can access all relevant information from the memory 110 of the computing device 104 which is a part of the hospital information system 102 over the network 116 to do the analysis to predict patient behavior. In some embodiments, the patient support device is connected to a bedside connector that accesses the relevant information for patient behavior analysis over the network 116.

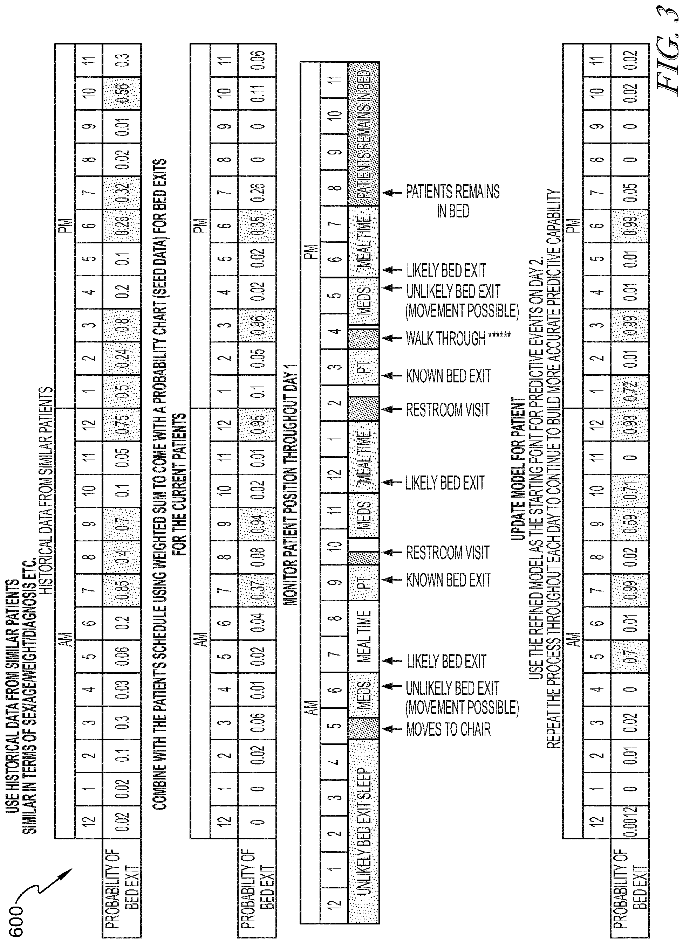

In the illustrated embodiment Fig. 2, a flowchart shows the different steps performed to determine a patient behavior. The processor 304 of a patient support apparatus 122 such as bed, can access historical data of similar patients (block 502) from memory 306 or from the memory 110 over a wireless network 116 at step 500. The current patient's information such as medical schedule, routine, visiting hours (block 510), and mobility information (bock 504) can be accessed from memory 306 or from memory 110 over a wireless network 116 at step 500. At step 512, the processor 304 of a patient support apparatus 122 accesses memory 306 or the memory 110 over a wireless network 116 to decide it is the first assessment of the day. If it is the first assessment, steps 514, 516 and 520 are executed prior to step 522, else step 518 is executed prior to step 522. At step 518, the processor 304 accesses the model developed for the current patient that is stored in memory 306 or from memory 110 over a wireless network 116. At step 514, weighted sum is used by the processor 306 to determine a probability chart for the current patient's behavior based on the information obtained in step 500. An illustrative probability chart 600, is shown in Fig. 3. At step 516, a model to predict current patient's behavior is developed using machine learning (ML) techniques such as supervised learning or reinforcement learning. This model can be used to predict the current patient's behavior. In some embodiments, this behavior is bed exits. At step 520, the model developed is stored in memory 306 or in memory 110 over a wireless network 116 or in both. At step 522, the model developed is used to make predictions about the current patient's bed exit behavior. If the patient is predicted to exit the bed, the prediction is communicated to the caregiver over the wireless network 116 at step 524 and added to the historical database at step 526. If the patient is not predicted to exit the bed, caregiver is not contacted and the information is added to the historical database at step 522. The processor can access mobility and scheduling information of the patient from various patient support apparatuses 112 controller 302 over the wireless network 116. In another embodiment, all the processing of information is done by the processor 106 in the computing device 104 which is a part of the hospital information system 102. The memory processor 106 can access all relevant information from memory 110 and memory 306 over the network 116.

The current patient's potential bed exits are monitored on Day 1 of the current patient's stay in the hospital. Any changes to the initial assumptions based on the current patients schedule and the historical data of similar patients is monitored. Machine learning methods such as supervised learning or reinforcement learning is used to update the probability of bed exit and update the model making predictions. This builds a more accurate characterization of the patient's behavior. The refined model is used as the starting point for predictive events on Day 2. This process is repeated each day that the current patient is in the hospital room.

In some embodiments, patient diagnosis is entered as an input called patient condition to the model at the user interface 324 on Day 1. Diagnosis is used to predict how the model will change over time. For example. A less critical diagnosis would expect the patient to become more mobile over time, a more critical diagnosis may expect the patient to deteriorate over time. The patient condition is used to update the probability of bed exit and update the model making predictions each day. This builds a more accurate characterization of the patient's behavior. The refined model is used as the starting point for predictive events on Day 2. This process is repeated each day that the current patient is in the hospital room. The current patient's data is integrated into historical data for use with next similar category patient and stored in the memory 306 of the patient support apparatus and also transmitted to the hospital information system 102 to be stored in the memory 110 and used by the processor 106.

The analysis done is used to provide alerts to the caregivers based on predicted events. Such alerts are proactive and planned care is provided to the patients based on the probability of the events that are likely to occur. These alerts are organized to reduce the cognitive burden by informing the caregivers about the most active time and are timed to reduce the occurrence of fall events. Such alerts are timed to improve patient safety and potential hospital liability and to increase staff effectiveness.

Using the data, the system can observe and predict future bed exit events to alert caregivers before the event occurs. That is, the system uses the data to track patient movement throughout the patient's stay in the healthcare facility. The system pinpoints the patient's position in the patient room and determines what time the patient is typically in bed, when the patient uses the restroom, when the patient eats lunch, and when the patient generally is not in the patient bed. These behavioral patterns are utilized to predict when the patient may exit the bed. For example, the system may determine that the patient generally wakes up at the same time each day and build a customized rounding schedule to check on the patient 10-20 minutes before they usually wake up. In another example, the system may determine that the patient uses the restroom an average of 60 minutes after eating and proactively alert a caregiver that the patient may need assistance after eating. In yet another example, the system may determine that the patient exits the patient bed for an average of two hours. Using this data, caregivers may be alerted to check on the patient after a predetermined time.

Although certain illustrative embodiments have been described in detail above, variations and modifications exist.

Embodiments of the invention can be described with reference to the following numbered clauses, with preferred features laid out in the dependent clauses:
1. A method for inferring a patient's future behavior, comprising:
   acquiring data related to patient bed exits,
   analyzing the data related to patient bed exits to detect patterns in patient bed exit behavior,
   building a customized schedule of patient bed exit behavior to predict future patient bed exits, and
   notifying a caregiver when a future patient bed exit is to occur.
2. The method of clause 1, wherein acquiring data related to patient bed exits includes acquiring data from a sensor in a patient support apparatus.
3. The method of either clause 1 or clause 2, wherein acquiring data related to patient bed exits includes acquiring data from a real time locating system.
4. The method of any preceding clause, wherein acquiring data related to patient bed exits includes acquiring data from a camera in a patient room.
5. The method of any preceding clause, wherein acquiring data related to patient bed exits includes acquiring data related to the patient's medical schedule.
6. The method of any preceding clause, wherein acquiring data related to patient bed exits includes acquiring data related to the patient's mealtime schedule.
7. The method of any preceding clause, wherein acquiring data related to patient bed exits includes acquiring data related to the patient's visiting hours schedule.
8. The method of any preceding clause, further comprising combining historical data from similar patients to the data related to patient bed exits to determine a model that predicts future patient bed exits.
9. The method of any preceding clause, wherein notifying a caregiver when a future patient bed exit is to occur further comprises notifying a caregiver a predetermined time before the future patient bed exit is to occur.
10. The method of any preceding clause, further comprising comparing a patient's current behavior to a patient's predicted behavior.
11. The method of clause 10, further comprising updating the customized schedule based on differences between a patient's current behavior and a patient's predicted behavior.
12. The method of any preceding clause, wherein notifying a caregiver when a future patient bed exit is to occur further comprises notifying the caregiver before the patient wakes up.
13. The method of any preceding clause, wherein notifying a caregiver when a future patient bed exit is to occur further comprises notifying the caregiver before the patient uses the restroom.
14. A system for inferring a patient's future behavior, comprising:
   a patient support apparatus,
   a data acquisition system to track data related to patient bed exits from the patient support apparatus, and
   a controller in communication with the patient support apparatus, the controller including a processor and a non-transitory memory device, the memory device including instructions that, when executed by the processor, acquire data related to patient bed exits, analyze the data related to patient bed exits to detect patterns in patient bed exit behavior, build a customized schedule of patient bed exit behavior to predict future patient bed exits, and notify a caregiver when a future patient bed exit is to occur.
15. The system of clause 14, wherein the data acquisition system further comprises a sensor in a patient support apparatus.
16. The system of either clause 14 or clause 15, wherein the data acquisition system further comprises a real time locating system.
17. The system of any one of clauses 14 to 16, wherein the data acquisition system further comprises a camera in a patient room.
18. The system of any one of clauses 14 to 17, wherein the data related to patient bed exits includes data related to the patient's medical schedule.
19. The system of any one of clauses 14 to 18, wherein the data related to patient bed exits includes data related to the patient's mealtime schedule.
20. The system of any one of clauses 14 to 19, wherein the data related to patient bed exits includes data related to the patient's visiting hours schedule.
21. The system of any one of clauses 14 to 20, wherein historical data from similar patients is compared to the data related to patient bed exits to determine a model that predicts future patient bed exits.
22. The system of any one of clauses 14 to 21, wherein the caregiver is notified a predetermined time before the future patient bed exit is to occur.
23. The system of any one of clauses 14 to 22, wherein a patient's current behavior is compared to a patient's predicted behavior to update the customized schedule.
24. The system of any one of clauses 14 to 23, wherein the caregiver is notified before the patient wakes up.
25. The system of any one of clauses 14 to 24, wherein the caregiver is notified before the patient uses the restroom.

## Claims

1. A system for inferring a patient's future behavior, comprising:
a patient support apparatus configured to support a patient,
a data acquisition system in communication with the patient support apparatus via a network, the data acquisition system configured to automatically track data related to patient bed exits from the patient support apparatus, wherein the data acquisition system includes at least one of a sensor to detect movement of the patient on the patient support apparatus, a real time locating system to detect a location of the patient, and a camera to monitor the patient with time-stamped video,
a hospital information system in communication with the patient support apparatus via the network, the hospital information system including a memory that stores scheduling data related to the patient, wherein the scheduling data related to the patient includes at least one of a patient medical schedule, a patient mealtime schedule, and a patient visiting hour schedule, and
a controller in communication with the patient support apparatus, the controller including a processor and a non-transitory memory device, the memory device including instructions that, when executed by the processor, acquire the data related to patient bed exits from the data acquisition system, acquire the scheduling data related to the patient from the hospital information system, automatically analyze the data related to patient bed exits and the scheduling data related to the patient to detect patterns in patient bed exit behavior, automatically develop a model of patient bed exit behavior to predict future patient bed exits, automatically store the model in the hospital information system, automatically access the hospital information system to determine when a future patient bed exit is to occur, and automatically alert a caregiver over the network when the future patient bed exit is to occur.

2. The system of claim 1, wherein historical data from similar patients is automatically compared to the data related to patient bed exits to develop the model of patient bed exit behavior.

3. The system of either claim 1 or claim 2, wherein the caregiver is automatically alerted over the network a predetermined time before the future patient bed exit is to occur.

4. The system of any preceding claim, wherein a patient's current behavior is automatically compared to a patient's predicted behavior to update the model of patient bed exit behavior.

5. The system of any preceding claim, wherein the caregiver is automatically alerted over the network before the patient wakes up.

6. The system of any preceding claim, wherein the caregiver is automatically alerted over the network before the patient uses the restroom.
